(19) Europäisches Patentamt
European Patent Office
Office européen des brevets

(11) EP 0 614 986 B1

(12) **FASCICULE DE BREVET EUROPEEN**

(45) Date de publication et mention de la délivrance du brevet:
**21.07.1999 Bulletin 1999/29**

(51) Int Cl.[6]: **C12P 41/00**, C12P 17/12, C07D 211/60, C07D 211/62, C07D 401/04

(21) Numéro de dépôt: **94400381.3**

(22) Date de dépôt: **23.02.1994**

(54) **Dédoublement enzymatique de pipéridine-2-carboxylates d'alkyle**

Enzymatische Racemat-Spaltung von 2-Piperidin-alkyl-carboxylaten

Enzymatic racemate cleavage of 2-pipéridine alkylcarboxylates

(84) Etats contractants désignés:
**AT BE CH DE DK ES FR GB GR IE IT LI LU MC NL PT SE**

(30) Priorité: **03.03.1993 FR 9302434**

(43) Date de publication de la demande:
**14.09.1994 Bulletin 1994/37**

(73) Titulaire: **SYNTHELABO**
**92350 Le Plessis Robinson (FR)**

(72) Inventeurs:
- **Perard, Serge**
  **F-94130 Nogent Sur Marne (FR)**
- **Zard, Lydia**
  **F-91190 Gif Sur Yvette (FR)**
- **Rossey, Guy**
  **F-78960 Voisins Le Bretonneux (FR)**

(74) Mandataire: **Thouret-Lemaitre, Elisabeth et al**
**Sanofi-Synthélabo**
**Service Brevets**
**174, avenue de France**
**75013 Paris (FR)**

(56) Documents cités:
**EP-A- 0 008 746** **EP-A- 0 474 129**
**WO-A-85/00599**

- **BULLETIN OF THE CHEMICAL SOCIETY OF JAPAN vol. 64, no. 11 , 1991 , TOKYO JP pages 3251 - 3255 TADASHI SHIRAIWA ET AL. 'Asymmetric transformations of proline and 2-piperidinecarboxylic acid via formation of salts with optically active tartaric acid.'**
- **TETRAHEDRON: ASYMMETRY vol. 3, no. 8 , 1992 , OXFORD GB pages 1021 - 1024 ROBERT CHENEVERT ET AL. 'Enzyme-catalysed hydrolysis of N-benzyloxycarbonyl-cis-2,6-(acetoxymethyl)piperidine.A facile route to optically active piperidines.'**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 614 986 B1

## Description

La présente invention a pour objet un procédé de préparation enzymatique de *(2R-trans)-pipéridine-2-carboxylates* d'alkyle optiquement purs de formule (1)

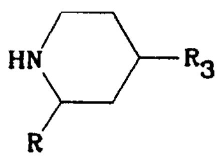

(1)

dans laquelle

R représente un groupe $(C_1-C_4)$alcoxycarbonyle et
$R_3$ représente un groupe $(C_1-C_4)$alkyle.

Ces composés sont des intermédiaires de synthèse de composés à activité antithrombotique.

Le 4-méthylpipéridine-2-carboxylate d'éthyle est décrit dans le brevet européen 0008746 sous forme de racémate et la préparation de la forme (2*R-trans*) est réalisée par cristallisation préférentielle dans l'acide tartrique via l'acide correspondant (acide 4-méthylpipéridine-2-carboxylique). R. Chênevert et coll. décrit, dans *Tetrahedron Asymmetry* (3, No 8, pp. 1021-1024 (1992), l'hydrolyse enzymatique de la *N*-benzyloxycarbonyle-*cis*-2,6-(acétoxyméthyl) pipéridine par une lipase isolée de *Aspergillus niger.*
Le procédé selon l'invention consiste en une séparation des énantiomères au moyen d'une enzyme capable d'hydrolyser préférentiellement l'énantiomère (2*S-trans*) en laissant intact l'énantiomère (2*R-trans*).
Le procédé selon l'invention permet d'obtenir l'énantiomère (2*R-trans*) optiquement pur, avec un bon rendement et des excès énantiomériques compris entre 98 et 99 %.

Le composé de départ, sous forme de racémate et de configuration trans, est dissous dans un solvant organique tel que par exemple le (1,1-diméthyléthyl)méthyléther, auquel on ajoute une solution aqueuse tamponnée par un mélange d'hydrogéno-phosphate de sodium et de dihydrogénophosphate de potassium 0,01 M à pH 7. On ajuste le pH du milieu réactionnel à 7,4 et on ajoute l'enzyme. La réaction s'effectue en 4 à 25 heures, selon l'enzyme utilisée, à une température de 20 à 37 °C et à un pH de 7,4 maintenu constant par addition d'une solution d'hydroxyde de sodium 1 M à l'aide d'un pH-stat. On contrôle l'avancement de la réaction par chromatographie liquide haute performance sur phase chirale. A la fin de la réaction on ajuste le pH à 9, on filtre l'insoluble, si nécessaire, et on extrait par un solvant organique tel que par exemple l'éther diéthylique.

Selon l'invention les enzymes à utiliser pour hydrolyser préférentiellement l'énantiomère (2*S*-trans) sont la lipase AY de *Candida rugosa* (Amano Pharmaceutical Ltd.), la lipase N de *Rhizopus* (Amano Pharmaceutical Ltd.), la lipase *Mucor* (Amano Pharmaceutical Ltd.) ou le prozyme 6 *Aspergillus* (Amano Pharmaceutical Ltd.), la protéase *Aspergillus oryzae* (Sigma), la poudre acétonique de pancréas de mouton (Sigma) ou la poudre acétonique de pancréas de porc type I (Sigma).

Les composés de départ sont disponibles dans le commerce ou décrits dans la littérature ou peuvent être préparés selon des méthodes qui y sont décrites ou qui sont connues de l'homme du métier.

Les exemples suivants illustrent le procédé de l'invention. Les microanalyses élémentaires et les spectres IR et RMN confirment la structure des composés obtenus.

Exemple 1

(2*R*- *trans*)-4-méthylpipéridine-2-carboxylate d'éthyle

*Méthode 1*

On dissout 10 g (58,5 mmoles) du racémate (*trans*)-4-méthylpipéridine-2-carboxylate d'éthyle dans 40 ml de (1,1-diméthyléthyl)méthyléther et 150 ml de tampon phosphate ($KH_2PO_4$ + $Na_2HPO_4$) 0,01 M à pH 7. On ajuste le pH à 7,4 avec une solution 1 M d'acide chlorhydrique puis on ajoute 8 g de poudre acétonique de pancréas de porc type I (Sigma). On maintient le pH constant pendant les 4 heures 30 de réaction par addition d'une solution d'hydroxyde de sodium 1 M à l'aide d'un pH-stat et on suit l'évolution de la réaction par chromatographie liquide haute performance sur phase chirale. Après addition de 31 ml d'hydroxyde de sodium, on ajoute 100 ml d'éther diéthylique, on ajuste le

pH à 9 et on filtre sur célite. On renouvelle l'extraction 4 fois, on rassemble les phases organiques, on les sèche sur du sulfate de magnésium, on les filtre et on évapore sous vide.
On obtient 4,23 g du composé attendu, sous forme d'une huile, avec un excès énantiomérique de 98%.
Rendement = 42 %
$[\alpha]_D^{22}$ = - 19 ° (c = 2,66; éthanol)

*Méthode 2*

On met en suspension 1,21 g (7,08 mmoles) du racémate (*trans*)-4-méthylpipéridine-2-carboxylate d'éthyle dans 30 ml de tampon phosphate ($KH_2PO_4$ + $Na_2HPO_4$) 0,01 M à pH 7. On ajuste le pH à 7 avec une solution 1 M d'acide chlorhydrique puis on ajoute 1,5 g de Prozyme 6 (Amano). On maintient le pH constant pendant les 22 heures de réaction par addition d'une solution d'hydroxyde de sodium 1 M à l'aide d'un pH-stat et on suit l'évolution de la réaction par chromatographie liquide haute performance sur phase chirale. Après 22 heures, on ajuste le pH à 9 et on extrait l'ester chiral par 4 fois 40 ml d'éther éthylique. On réunit les phases organiques, on les sèche sur du sulfate de magnésium, on filtre et on évapore sous vide.
On obtient 430 mg du composé attendu, sous forme d'une huile, avec un excès énantiomérique de 96%.
Rendement = 35 %

Exemple 2

(2*R*-*trans*)-4-éthylpipéridine-2-carboxylate d'éthyle

On solubilise 1 g (5,4 mmoles) du racémate (*trans*)-4-éthylpipéridine-2-carboxylate d'éthyle dans 58 ml de toluène. On ajoute 58 ml de tampon phosphate ($KH_2PO_4$ + $Na_2HPO_4$) 0,01 M à pH 7 et 1,3 g de poudre acétonique de pancréas de porc type I (Sigma). On suit l'évolution de la réaction par chromatographie liquide haute performance sur colonne chirale et au bout de 4 heures, on ajoute 50 ml d'éther diéthylique, on ajuste 4 fois, on rassemble les phases organiques, on les sèche sur du sulfate de magnésium, on les filtre et on évapore sous vide.
On obtient 500 mg de produit, sous forme d'une huile, avec un excès énantiomérique de 99 %.
Rendement = 50 %
$[\alpha]_D^{20}$ = - 13,45 ° (c = 1,13; chloroforme)
Les dérivés de formule (1) obtenus selon le procédé de l'invention, sont utiles pour la synthèse de trifluoroacétates de formule (2)

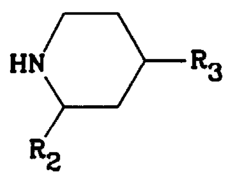

, $CF_3COOH$

(2)

dans laquelle

$R_2$ repésente soit un groupe carboxylique, soit un groupe carboxylate de sodium, soit un groupe amide de formule $-CONR_4R_5$, soit un groupe $-CH_2OR_4$, soit un groupe $-CN_4R_4$, $R_4$ étant un atome d'hydrogène ou un groupe ($C_1$-$C_4$)alkyle et $R_5$ étant un atome d'hydrogène, un groupe ($C_1$-$C_4$)alkyle, un groupe hydroxy, un groupe ($C_1$-$C_4$)alcoxy ou un groupe phénylméthoxy et
$R_3$ représente un groupe ($C_1$-$C_4$)alkyle,

sous la forme (2*R*-*trans*).
Ainsi les composés de formule (2) dans laquelle $R_2$ représente un groupe $-CONR_4R_5$ où $R_4$ est un atome d'hydrogène ou un groupe ($C_1$-$C_4$)alkyle et $R_5$ est un atome d'hydrogène, un groupe ($C_1$-$C_4$)alkyle, un groupe hydroxy, un groupe ($C_1$-$C_4$)alcoxy ou un groupe phénylméthoxy peuvent être préparés de la manière sui-vante : on traite un composé de formule (1) par du dicarbo-nate de bis(1,1-diméthyléthyle), dans un solvant tel que le dichlorométhane, dans des conditions classiques. On obtient un composé protégé sur l'azote, que l'on saponifie par une solution aqueuse d'hydroxyde de sodium puis on fait réagir une amine de formule $HNR_4R_5$, dans laquelle $R_4$ et $R_5$ sont tels que définis précédemment, pour obtenir un amide; la réaction est réalisée en présence d'une base comme par exemple la 4-méthylmorpholine ou

la N,N-diisopropyléthylamine (base de "Hunig") et d'un agent couplant tel que par exemple l'hexafluorophosphate de [(benzotriazol-1-yl)oxy]tris (diméthylamino)phosphonium ou l'isobutylchloroformiate. Ensuite, on fait réagir sur l'amide obtenu, l'acide trifluoroacétique dans le dichlorométhane, éventuellement en présence de méthoxybenzène.

Les composés de formule (2) dans laquelle $R_2$ représente un groupe $-CH_2OR_4$ où $R_4$ est un atome d'hydrogène peuvent être synthétisés de la manière suivante : on traite un composé de formule (1) par du dicarbonate de bis (1,1-diméthyléthyle) dans un solvant tel que le dichlorométhane dans des conditions classiques. On obtient un composé protégé sur l'azote, que l'on saponifie par une solution aqueuse d'hydroxyde de sodium puis on réduit la fonction carboxyle, soit par la méthode à l'anhydride mixte décrite par Valerio R.M. et coll. dans Synthesis, 1988, 786, soit par l'action du diméthylsulfure de borane dans un solvant tel que le tétrahydrofurane. Ensuite, on déprotège l'amine par action de l'acide trifluoroacétique.

Les composés de formule (2) dans laquelle $R_2$ représente un groupe $-CH_2OR_4$ où $R_4$ est un groupe $(C_1-C_4)$ alkyle peuvent être synthétisés de la manière suivante : on traite un composé de formule (1) par du dicarbonate de bis(1,1-diméthyléthyle) dans un solvant tel que le dichlorométhane dans des conditions classiques. On obtient un composé protégé sur l'azote, que l'on saponifie par une solution aqueuse d'hydroxyde de sodium; ensuite on fait réagir un halogénure d'alkyle, de préférence un iodure d'alkyle, en présence d'une base telle que par exemple l'hydrure de sodium, puis on traite le composé ainsi obtenu par l'acide trifluoroacétique dans le dichlorométhane.

Les composés de formule (2) dans laquelle $R_2$ représente un groupe $-CN_4R_4$ dans lequel $R_4$ est un atome d'hydrogène peuvent être synthétisés de la manière suivante : on traite un composé de formule (1) par du dicarbonate de bis(1,1-diméthyléthyle) dans un solvant tel que le dichlorométhane dans des conditions classiques. On obtient un composé protégé sur l'azote, que l'on saponifie par une solution aqueuse d'hydroxyde de sodium. On condense le composé ainsi obtenu avec de l'ammoniaque, en présence d'une base comme par exemple la 4-méthylmorpholine et d'un agent couplant tel que par exemple l'isobutylchloroformiate dans un solvant tel que le tétrahydrofurane. On déshydrate le carboxamide ainsi obtenu par de l'oxytrichlorure de phosphore, pour obtenir un nitrile que l'on chauffe en présence d'azoture de sodium et de chlorure d'ammonium dans un solvant tel que par exemple le diméthylformamide. On obtient un dérivé tétrazolyle que l'on traite par l'acide trifluoroacétique.

Les composés de formule (2) dans laquelle $R_2$ représente un groupe $-CN_4R_4$ dans lequel $R_4$ est un groupe $(C_1-C_4)$alkyle peuvent être synthétisés de la manière suivante : on traite un composé de formule (1) par du dicarbonate de bis(1,1-diméthyléthyle) dans un solvant tel que le dichlorométhane dans des conditions classiques. On obtient un composé protégé sur l'azote, que l'on saponifie par une solution aqueuse d'hydroxyde de sodium. On condense le composé ainsi obtenu avec de l'ammoniaque, en présence d'une base comme par exemple la 4-méthylmorpholine et d'un agent couplant tel que par exemple l'isobutylchloroformiate dans un solvant tel que le tétrahydrofurane. On déshydrate le carboxamide ainsi obtenu par de l'oxytrichlorure de phosphore, pour obtenir un nitrile que l'on chauffe en présence d'azoture de sodium et de chlorure d'ammonium dans un solvant tel que par exemple le diméthylformamide. Ensuite on condense le dérivé tétrazolyle obtenu avec un halogénure d'alkyle, de préférence un iodure d'alkyle puis on traite le composé obtenu par l'acide trifluoroacétique.

Les exemples 3 à 5 qui suivent illustrent l'utilisation des composés de formule (1) pour la synthèse de composés de formule (2).

<u>Exemple 3</u>

trifluoroacétate de (2*R*-*trans*)-*N*-éthyl-4-méthylpipéridine-2-carboxamide

3.1. ester 1-(1,1-diméthyléthylique) de l'acide (2*R*-*trans*)-4-méthylpipéridine-1,2-dicarboxylique

On place 510 mg (3 mmoles) de (2*R*-*trans*)-4-méthylpipéridine-2-carboxylate d'éthyle dans 10 ml de dichlorométhane et on ajoute, sous azote, à 0 °C, 654 mg (3 mmoles) de dicarbonate de bis(1,1-diméthyléthyle). On laisse remonter la température du milieu réactionnel à la température ambiante pendant une nuit, on l'évapore et on reprend le résidu dans 4 ml d'éthanol et 3 ml d'une solution d'hydroxyde de sodium 1 N. On laisse le mélange une nuit à la température ambiante puis on ajoute de nouveau 3 ml d'une solution d'hydroxyde de sodium 1 N. Ensuite, on évapore le milieu réactionnel, on reprend le résidu par de l'eau et on le lave avec de l'éther. On acidifie le milieu avec de l'acide chlorhydrique 1 N puis on l'extrait deux fois par de l'éther. On rince les phases organiques par 10 ml d'une solution saturée en chlorure de sodium et on les sèche sur du sulfate de magnésium.
On obtient 0,68 g de produit.
Point de fusion = 87-90 °C     Rendement = 97 %

3.2. (2*R*-*trans*)-2-[(éthylamino)carbonyl]-4-méthylpipéridine-1-carboxylate de 1,1-diméthyléthyle

On dissout 1 (4,11 mmoles) de l'ester 1-(1,1-diméthyléthylique) de l'acide (2*R*-*trans*)-4-méthylpipéridine-1,2-dicarboxylique dans 15 ml de tétrahydrofurane. Sous argon et à la température ambiante, on ajoute 0,45 ml (4 mmoles) de 4-méthylmorpholine puis on refroidit à une température comprise entre -10 et -15 °C. On ajoute ensuite 0,53 ml (4 mmoles) de chloroformate de 2-méthylpropyle puis 0,278 g (6 mmoles) d'éthanamine en solution dans 3 ml de tétrahydrofurane. On laisse remonter le milieu à la température ambiante puis on évapore le tétrahydrofurane. On reprend le résidu par 100 ml d'éther et on le lave successivement avec 50 ml d'acide chlorhydrique 1 N, 50 ml d'eau et finalement 50 ml d'une solution saturée en hydrogénocarbonate de sodium. On le sèche sur du sulfate de magnésium.
On obtient 1,06 g de produit.
Rendement = 95 %

3.3. trifluoroacétate de (2*R-trans*)-N-éthyl-4-méthylpipéridine-2-carboxamide

On met en solution dans 5,4 ml de dichlorométhane, 0,352 g (1,3 mmoles) de (2*R-trans*)-2-[(éthylamino) carbonyl]-4-méthylpipéridine-1-carboxylate de 1,1-diméthyléthyle. Sous argon et à 0 °C, on ajoute 5,4 ml d'acide trifluoroacétique. On laisse le milieu remonter à la température ambiante puis on l'évapore. On reprend le résidu dans 50 ml de dichlorométhane et on évapore de nouveau.
On obtient 0,686 g de produit brut.

<u>Exemple 4</u>

trifluoroacétate de (2*R-trans*)-4-méthyl-N-(phénylméthoxy)pipéridine-2-carboxamide

4.1. (2*R-trans*)-4-méthyl-2-[[(phénylméthoxy)amino]carbonyl]pipéridine-1-carboxylate de 1,1-diméthyléthyle

Sous azote, on place 1 g (4,03 mmoles) de l'ester 1-(1,1-diméthyléthylique) de l'acide (2*R-trans*)-4-méthylpipéridine-1,2-dicarboxylique dans 15 ml de tétrahydrofurane. On refroidit à -15 °C et on ajoute 0,47 ml (4 mmoles) de 4-méthylmorpholine et 0,56 ml (4 mmoles) de chloroformate de 2-méthylpropyle. On agite pendant 5 minutes et on ajoute 0,755 g (4,7 mmoles) de chlorhydrate de *O*-(phénylméthyl)hydroxylamine, en solution dans un mélange de 5 ml de tétrahydrofurane et de 0,52 ml de 4-méthylmorpholine. On agite pendant une nuit à la température ambiante puis on verse le milieu réactionnel sur 3 volumes d'acétate d'éthyle. On le lave successivement par 50 ml d'acide chlorhydrique 0,1 N, 50 ml d'eau, 50 ml d'hydrogénocarbonate de sodium, 50 ml d'eau et finalement par 50 ml d'une solution saturée en chlorure de sodium.
On le sèche sur du sulfate de magnésium et on recristallise le résidu par trituration dans l'éther.
On obtient 0,95 g de produit.
Point de fusion = 107-108 °C

4.2. trifluoroacétate de (2*R-trans*)-4-méthyl-*N*-(phénylméthoxy)pipéridine-2-carboxamide

On agite pendant 15 minutes, à 0 °C, une solution de 450 mg (1,4 mmoles) de (2*R-trans*)-4-méthyl-2-[[(phénylméthoxy)amino]carbonyl]pipéridine-1-carboxylate de 1,1-diméthyléthyle, dans un mélange de 5 ml d'acide trifluoroacétique et de 5 ml de dichlorométhane. On évapore, on reprend le résidu par 10 ml de benzène et on évapore de nouveau.
On obtient 700 mg de produit sous forme d'une huile.

<u>Exemple 5</u>

trifluoroacétate de (2*R-trans*)-4-méthyl-2-(1*H*-tétrazol-5-yl) pipéridine

5.1. (2*R-trans*)-2-(aminocarbonyl)-4-méthylpipéridine-1-carboxylate de 1,1-diméthyléthyle

Sous azote, on place 1 (4,03 mmoles) de l'ester 1-(1,1-diméthyléthylique) de l'acide (2*R-trans)*-4 -méthylpipéridine-1,2-dicarboxylique dans 15 ml de tétrahydrofurane. On refroidit le milieu réactionnel à -15 °C et on ajoute 0,47 ml (4 mmoles) de 4-méthylmorpholine puis 0,56 ml (4 mmoles) de chloroformate de 2-méthylpropyle. On agite le mélange pendant 5 minutes puis on ajoute en excès de l'ammoniaque. On laisse remonter à la température ambiante puis on agite le milieu réactionnel pendant 1 heure. On le verse sur 3 volumes d'acétate d'éthyle et on le lave successivement par 50 ml d'acide chlorhydrique 0,1 N, 50 ml d'eau, 50 ml d'une solution d'hydrogénocarbonate de sodium, 50 ml d'eau et finalement par 50 ml d'une solution saturée en chlorure de sodium. On le sèche sur du sulfate de magnésium et on l'évapore.

On obtient 1 g de produit sous forme d'une huile opaque que l'on utilise telle quelle dans l'étape suivante.

5.2. (2*R-trans*)-2-cyano-4-méthylpipéridine-1-carboxylate de 1,1-diméthyléthyle

On reprend 1 g (4 mmoles) de (2*R-trans*)-2-(aminocarbonyl)-4-méthylpipéridine-1-carboxylate de 1,1-diméthyléthyle dans 5 ml de pyridine, on refroidit à -5 °C et on ajoute goutte à goutte, 0,52 ml de chlorure de phosphoryle dans 1 ml de dichlorométhane. On agite le milieu réactionnel pendant 1 heure puis on le verse sur de la glace et on l'extrait par 100 ml d'éther diéthylique. On l'évapore et on purifie le résidu par chromatographie sur colonne de gel de silice en éluant par du dichlorométhane.
On obtient 0,7 g de produit sous forme d'une huile que l'on utilise telle quelle dans l'étape suivante.

5.3. (2*R-trans*)-4-méthyl-2-(1*H*-tétrazol-5-yl)pipéridine

Dans un tube scellé, on place 0,7 g (3,1 mmoles) de (2*R-trans*)-2-cyano-4-méthylpipéridine-1-carboxylate de 1,1-diméthyléthyle et on ajoute 1,5 ml de diméthylformamide, 212 mg (3 mmoles) d'azoture de sodium et 180 mg (4 mmoles) de chlorure d'ammonium. On chauffe le milieu réactionnel à 100 °C pendant 24 heures puis on l'évapore. On reprend le résidu dans un mélange de carbonate de sodium et d'éther diéthylique, on recueille la phase organique, on ajuste le pH à 2 avec de l'acide chlorhydrique 1 N et on l'extrait par de l'éther diéthylique. On récupère la phase organique, on la sèche sur du sulfate de magnésium et on l'évapore.
On obtient 0,3 g de produit sous forme d'une huile que l'on utilise telle quelle dans l'étape suivante.

5.4. trifluoroacétate de (2*R-trans*)-4-méthyl-2-(1*H*-tétrazol-5-yl)pipéridine

On dissout, sous azote, 242 mg (0,9 mmole) de (2*R-trans*)-4-méthyl-2-(1*H*-tétrazol-5-yl)pipéridine dans 2 ml de dichlorométhane et on ajoute 2 ml d'acide trifluoroacétique. On agite pendant 1 heure à la température ambiante, on évapore et on reprend le résidu par du dichlorométhane. On évapore à nouveau.
On obtient 355 mg de produit sous forme d'une huile.
Les composés de formules (1) et (2) sont utiles pour la synthèse de composés à activité antithrombotique tels que ceux décrits dans le brevet européen no 0008746, comme par exemple l'argatroban, et pour celle des composés décrits dans la demande de brevet européen no 0565396, composés dont la structure comporte une pipéridine de formule (3)

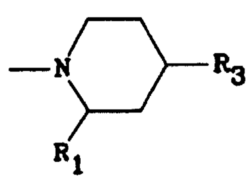

(3)

dans laquelle

$R_1$ représente soit un groupe $(C_1\text{-}C_4)$alcoxycarbonyle, soit un groupe carboxylique, soit un groupe carboxylate de sodium, soit un groupe amide de formule $-CONR_4R_5$, soit un groupe $-CH_2OR_4$, soit un groupe $-CN_4R_4$, $R_4$ étant un atome d'hydrogène ou un groupe $(C_1\text{-}C_4)$alkyle et $R_5$ étant un atome d'hydrogène, un groupe $(C_1\text{-}C_4)$alkyle, un groupe hydroxy, un groupe $(C_1\text{-}C_4)$alcoxy ou un groupe phénylméthoxy et
$R_3$ représente un groupe $(C_1\text{-}C_4)$alkyle,

sous la forme (2*R-trans*).
Les exemples 6 et 7 qui suivent illustrent la synthèse de composés décrits dans la demande de brevet européen no 0565396 à partir des composés de formule (1) ou (2).

Exemple 6

chlorhydrate de (2*R-trans*)-N-éthyl-1-[5-(1*H*-imidazol-4-yl)-2-[[(3-méthyl-1,2,3,4-tétrahydroquinoléin-8-yl)sulfonyl]amino]-1-oxopentyl]-4-méthylpipéridine-2-carboxamide

6.1. (2*R-trans*)-*N*-éthyl-4-méthyl-1-[2-[[(3-méthyl-1,2,3,4-tétrahydroquinoléin-8-yl)sulfonyl]amino]-1-oxo-5-[1-(triphénylméthyl)-1*H*-imidazol-4-yl]pentylpipéridine-2-carboxamide

On met en solution 0,221 g (1,3 mmoles) de trifluoroacétate de (2*R-trans*)-*N*-éthyl-4-méthylpipéridine-2-carboxamide obtenu selon la méthode décrite dans l'exemple 3, dans 10 ml de selon la méthode décrite dans l'exemple 3, dans 10 ml de dichlorométhane. Sous argon et à 0 °C, on ajoute 0,635 g (1 mmole) d'acide (*S*)-α-[[(3-méthyl-1,2,3,4-tétrahydroquinoléin-8-yl)sulfonyl]amino]-1-(triphénylméthyl)-1*H*-imidazole-4-pentanoïque obtenu selon la méthode décrite dans la demande de brevet européen no 0565396, 0,486 g (1,10 mmoles) d'hexafluorophosphate de [(benzotriazol-1-yl)oxy]tris(diméthylamino)phosphonium et 1,04 ml (6 mmoles) de N,N-diisopropyléthylamine. On laisse le mélange remonter à la température ambiante pendant une nuit puis on le lave successivement par 10 ml d'acide chlorhydrique 1 N et par 10 ml d'une solution saturée en hydrogénocarbonate de sodium. On le sèche sur du sulfate de magnésium.
On obtient 0,337 g de produit.
Point de fusion = 30-40°C Rendement = 43 %

6.2. chlorhydrate de (2*R-trans*)-*N*-éthyl-1-[5-(1*H*-imidazol-4-yl)-2-[[(3-méthyl-1,2,3,4-tétrahydroquinoléin-8-yl) sulfonyl]amino]-l-oxopentyl]-4-méthylpipéridine-2-carboxamide

On solubilise 0,332 g (0,422 mmole) de (2*R-trans*)-*N*-éthyl-4-méthyl-1-[2-[[(3-méthyl-1,2,3,4-tétrahydroquinoléin-8-yl)sulfonyl]amino]-l-oxo-5-[1-(triphénylméthyl)-1*H*-imidazol-4-yl]pentylpipéridine-2-carboxamide dans 3,7 ml d'acide acétique, puis on ajoute 0,7 ml d'eau et 3 ml de tétrahydrofurane. On chauffe le mélange au reflux pendant une heure. On évapore à sec et on purifie le résidu par chromatographie sur colonne de gel de silice en éluant par un gradient dichlorométhane/éthanol (90/10 à 85/15).
On obtient 0,225 g de produit sous forme de base.
On prépare le chlorhydrate en mettant 0,225 g de base en solution dans 4,1 ml d'acide chlorhydrique 0,1 N dans l'alcool isopropylique. On évapore et on reprend le résidu par de l'éther. On le filtre et on le sèche.
On obtient 0,200 g de chlorhydrate.
Point de fusion = 105-110 °C (décomposition)
$[\alpha]_D^{20}$ = + 33,2 (c = 0,2; méthanol)

Exemple 7

complexe de sodium du [2*R*-[1(*S*),2α,4β]]-4-méthyl-1-[2-[[(3-méthyl-1,2,3,4-tétrahydroquinoléin-8-yl)sulfonyl]amino]-1-oxo-5-(1*H*-imidazol-4-yl)pentyl]pipéridine-2-carboxylate de sodium

7.1. [2*R*-[1(*S*),2α,4β]]-4-méthyl-1-[2-[[(3-méthyl-1,2,3,4-tétrahydroquinoléin-8-yl)sulfonyl]amino]-1-oxo-5-(1*H*-imidazol-4-yl)pentyl]pipéridine-2-carboxylate d'éthyle

7.1.1. chlorure de triphényl[[(1-triphénylméthyl)-1*H*-imidazol-4-yl]méthyl]phosphonium

A 670 ml d'une solution de 105,5 g (294 mmoles) de 4-(chlorométhyl)-1-(triphénylméthyl)-1*H*-imidazole dans le diméthylformamide, on ajoute 77,7 g (296 mmoles) de triphénylphosphine. On chauffe à 80 °C pendant 3 heures. On évapore le solvant, on reprend le brut dans l'éther et on le triture. On filtre le précipité et on le sèche sous vide sur du pentoxyde de phosphore.
On obtient 162 g de produit sous forme de cristaux jaunâtres.
Point de fusion = 210 °C °C  Rendement 89 %

7.1.2. (*S,E*) -2-[[(phénylméthoxy)carbonyl]amino]-5-[1-(triphénylméthyl)-1*H*-imidazol-4-yl]pent-4-énoate de 1,1-diméthyléthyle

Dans un ballon tricol, sous argon, on introduit 50,93 g (820 mmoles) de chlorure de triphényl[[1-triphénylméthyl)-1*H*-imidazol-4-yl]méthyl]phosphonium en solution dans 333 ml de tétrahydrofurane. On ajoute goutte à goutte, à - 70 °C, 51,2 ml d'une solution 1,6 M de n-butyllithium dans l'hexane (820 mmoles). Après 30 minutes d'agitation à -

70 °C, on verse rapidement le milieu réactionnel dans 270 ml d'une solution 0,253 M, refroidie à 70 °C, de (*S*)-4-oxo-2-[[(phénylméthoxy)carbonyl]amino]butanoate de 1,1-diméthyléthyle dans le tétrahydrofurane (683 mmoles). On laisse remonter le mélange à la température ambiante pendant une nuit. On hydrolyse le mélange avec 280 ml d'une solution aqueuse saturée de chlorure de sodium. On sépare la phase aqueuse de la phase organique et on l'extrait par 2 fois 140 ml d'acétate d'éthyle. On réunit les phases organiques, on les sèche sur du sulfate de magnésium et on évapore à sec. On purifie le résidu par chromatographie sur colonne de gel de silice en éluant par un gradient hexane/acétate d'éthyle.
On obtient un mélange d'oléfine cis et trans.
Pour la forme cis:
Point de fusion - 66 °C $R_f$ = 0,30
[hexane/acétate d'éthyle (60/40)]
Pour la forme trans:
$R_f$ = 0,15 [hexane/acétate d'éthyle (60/40)]
Rendement = 40 %

7.1.3. chlorhydrate de (*S*)-2-amino-5-[1-(triphénylméthyl)-1*H*-imidazol-4-yl]pentanoate de 1,1-diméthyléthyle

On met en solution 5,83 g (9,50 mmoles) de (*S,E*)-2-[[(phénylméthoxy)carbonyl]amino]-5-[1-(triphénylméthyl)-1*H*-imidazol-4-yl]pent-4-énoate de 1,1-diméthyléthyle sous forme cis dans 120 ml d'éthanol. On réalise, pendant 5 heures, une hydrogénation catalytique, à 0,3 MPa, en présence de palladium sur charbon comme catalyseur. On filtre le catalyseur sur un mélange célite/silice et on évapore le solvant. On recueille 4,32 g de produit que l'on dissout dans 90 ml d'alcool isopropylique "chlorhydrique" (HCl = 0,1 N) chaud. On évapore le solvant, on précipite par de l'éther et on sèche le produit sous vide.
On obtient 3,62 g de produit.
Point de fusion = 73 °C Rendement = 73 t

7.1.4. (*S*)-2-[[(3-méthylquinoléin-8-yl)sulfonyl]amino]-5-[1-(triphénylméthyl)-1*H*-imidazol-4-yl]pentanoate de 1,1-diméthyléthyle

A 3,8 g (7,26 mmoles) de chlorhydrate de (*S*)-2-amino-5-[1-(triphénylméthyl)-1*H*-imidazol-4-yl]pentanoate de 1,1-diméthyléthyle, on ajoute 1,76 g (7,28 mmoles) de 8-(chlorosulfonyl)-3-méthylquinoléine en solution dans 50 ml de chloroforme, en présence de 2,1 ml (14,5 mmoles) de triéthylamine, à 5 °C. On laisse le milieu réactionnel sous agitation pendant 3 heures, on sépare la phase organique, on la lave par une solution d'acide chlorhydrique 0,1 N et on l'évapore. On purifie le brut par chromatographie sur colonne de gel de silice en éluant par un mélange éthanol/dichlorométhane (5/95).
On obtient 3,6 g de produit.
Point de fusion = 56 °C Rendement = 72 %

7.1.5. acide (*S*)-2-[[(3-méthylquinoléin-8-yl)sulfonyl]amino]-

5-[1-(triphénylméthyl)-1*H*-imidazol-4-yl]pentanoïque Dans une solution de 2,33 g (3,39 mmoles) de (*S*)-2-[[(3-méthylquinoléin-8-yl)sulfonyl]amino]-5-[1-(triphénylméthyl)-1*H*-imidazol-4-yl]pentanoate de 1,1-diméthyléthyle, dans 34 ml de benzène, refroidie à 0 °C, sous azote, on fait barboter, pendant 15 minutes, de l'acide chlorhydrique gazeux. On laisse le milieu réactionnel revenir à la température ambiante et on maintient l'agitation pendant 2 heures à cette température. On évapore le solvant sous vide et on purifie le résidu par chromatographie sur colonne de gel de silice en éluant par un mélange éthanol/dichlorométhane (20/80).
On obtient 1,42 g de produit sous forme d'une poudre blanchâtre.
Point de fusion = 170 °C Rendement = 66 %

7.1.6. [2*R*-[1(*S*),2α,4β]]-4-méthyl-1-[2-[[(3-méthylquinoléin-8-yl)sulfonyl]amino]-1-oxo-5-[1-(triphénylméthyl)-1*H*-imidazol-4-yl]pentyl]pipéridine-2-carboxylate d'éthyle

A une suspension de 2,23 g (3,53 mmoles) d'acide (*S*)-2-[[(3-méthylquinoléin-8-yl)sulfonyl]amino]-5-[1-(triphénylméthyl)-1*H*-imidazol-4-yl]pentanoïque dans 50 ml d'acétonitrile, on ajoute, à 0 °C, sous azote, 1,56 g (3,53 mmoles) d'hexafluorophosphate de [(benzotriazol-1-yl)oxy]tris(diméthylamino) phosphonium, 0,78 ml (7,06 mmoles) de 4-méthylmorpholine, 0,61 g (3,57 mmoles) de (2*R*-*trans*)-4-méthylpipéridine-2-carboxylate d'éthyle et 30 ml de dichlorométhane. On laisse, sous agitation, à la température ambiante, pendant 5 heures. On hydrolyse le milieu réactionnel par une solution saturée de chlorure de sodium et on l'extrait avec du chloroforme. On lave la phase

organique successivement par une solution d'acide chlorhydrique 0,1 N, par une solution saturée d'hydrogénocarbonate de sodium, par de l'eau et finalement par une solution de chlorure de sodium. On la sèche sur du sulfate de magnésium et on purifie le brut obtenu par chromatographie sur colonne de gel de silice en gluant par un mélange éthanol/dichlorométhane (5/95).
On obtient 1,94 g de produit.
$R_f$ = 0,68 (dichlorométhane/éthanol 95/5)
Rendement = 70 %

7.1.7. chlorhydrate de [2*R*-[1(*S*),2α,4β]]-4-méthyl-1-[2-[[(3-méthyl-1,2,3,4-tétrahydroquinoléin-8-yl)sulfonyl]amino]-1-oxo-5-(1*H*-imidazol-4-yl)pentyl]pipéridine-2-carboxylate d'éthyle

A 1,94 g (2,47 mmoles) de [2*R*-[1(*S*),2α,4β]]-4-méthyl-1-[2-[[(3-méthylquinoléin-8-yl)sulfonyl]amino]-1-oxo-5-[1-(triphénylméthyl)-1*H*-imidazol-4-yl]pentyl]pipéridine-2-carboxylate d'éthyle, on ajoute 70 ml d'éthanol et 17 ml d'acide acétique. On réalise une hydrogénation catalytique, en présence de palladium sur charbon, à 80 °C, pendant 6 heures. On filtre le mélange et on évapore le solvant. On reprend le résidu obtenu par de l'acide chlorhydrique 1 N, on le lave par de l'éther et on l'extrait par de acétate d'éthyle.
On obtient 1,05 g de produit.
Point de fusion = 104 °C (chlorhydrate)
Rendement = 78 %
$[\alpha]_D^{20}$ = + 101 (c = 0,2; méthanol)

7.2. complexe de sodium du [2*R*-[1(*S*),2α,4β]]-4-méthyl-1-[2-[[(3-méthyl-1,2,3,4-tétrahydroquinoléin-8-yl)sulfonyl] amino]-1-oxo-5-(1*H*-imidazol-4-yl)pentyl]pipéridine-2-carboxylate de sodium

A une solution de 0,201 g (0,4 mmole) de [2*R*-[1(*S*),2α,4β]]-4-méthyl-1-[2-[[(3-méthyl-1,2,3,4-tétrahydroquinoléin-8-yl)sulfonyl]amine]-1-oxo-5-(1*H*-imidazol-4-yl)pentyl]pipéridine-2-carboxylate d'éthyle, on ajoute, à la température ambiante, sous argon, 0,4 ml d'une solution de soude à 1 N titrée. On agite le milieu réactionnel à la température ambiante pendant 36 heures et on suit la réaction par chromatographie sur couche mince. Lorsque la réaction est terminée, on chauffe le milieu réactionnel à 55 °C pendant 7 heures. On laisse refroidir et on ajoute 1 ml d'éthanol et 50 ml d'éther. Après trituration, on obtient un précipité blanchâtre que l'on filtre et que l'on sèche.
On obtient 170 mg de produit sous forme d'une poudre blanche.
Point de fusion = 215 °C Rendement = 86 %
$[\alpha]_D^{20}$= + 67,2 (c = 0,1; méthanol)

## Revendications

**1.** Procédé de préparation de (2*R-trans*)-pipéridine-2-carboxylates d'alkyle de formule (1)

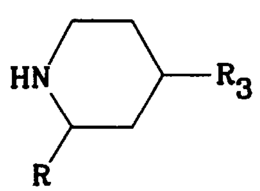

(1)

dans laquelle

R représente un groupe ($C_1$-$C_4$)alcoxycarbonyle et
$R_3$ représente un groupe ($C_1$-$C_4$)alkyle,

procédé caractérisé en ce que l'on fait réagir un pipéridine-2-carboxylate d'alkyle, sous forme de racémate de configuration trans, avec une enzyme qui dégrade spécifiquement l'énantiomère (2*S-trans*) en laissant sensiblement intact l'énantiomère (2*R-trans*).

**2.** Procédé selon la revendication 1, caractérisé en ce que l'enzyme est choisie parmi la lipase AY de *Candida rugosa*

(Amano Pharmaceutical Ltd.), la lipase N de *Rhizopus* (Amano Pharmaceutical Ltd.), la lipase *Mucor* (Amano Pharmaceutical Ltd.) ou le prozyme 6 *Aspergillus* (Amano Pharmaceutical Ltd.), la protéase *Aspergillus oryzae* (Sigma), la poudre acétonique de pancréas de mouton (Sigma) ou la poudre acétonique de pancréas de porc type I (Sigma).

**3.** Procédé selon l'une quelconque des revendications 1 et 2, caractérisé en ce que l'enzyme est le prozyme 6 *Aspergillus* (Amano Pharmaceutical Ltd.).

**4.** Procédé selon l'une quelconque des revendications 1 et 2, caractérisé en ce que l'enzyme est la poudre acétonique de pancréas de porc type I (Sigma).

**Patentansprüche**

**1.** Verfahren zur Herstellung von (2*R*-trans)-Piperidin-2-carbonsäurealkylester der Formel (1)

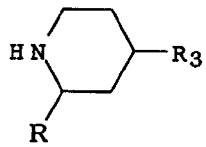

(1)

in der

R eine ($C_1$-$C_4$)-Alkoxycarbonylgruppe und
$R_3$ eine ($C_1$-$C_4$)-Alkylgruppe bedeuten,

dadurch gekennzeichnet, daß man einen Piperidin-2-carbonsäurealkylester in Form des Racemats in der trans-Konfiguration mit einem Enzym umsetzt, welches spezifisch das (2*S*-trans)-Enantiomere abbaut und das (2*R*-trans)-Enantiomere deutlich intakt läßt.

**2.** Verfahren nach Anspruch 1, dadurch gekennzeichnet, daß das Enzym ausgewählt ist aus Lipase AY von *Candida rugosa* (Amano Pharmaceutical Ltd. ), Lipase N von *Rhizopus* (Amano Pharmaceutical Ltd.), Lipase *Mucor* (Amano Pharmaceutical Ltd.) oder dem Prozym 6 *Aspergillus* (Amano Pharmaceutical Ltd.), Protease *Aspergillus oryzae* (Sigma), Acetonpulver vom Schafpankreas (Sigma) oder Acetonpulver vom Schweinzepankreas Typ I (Sigma).

**3.** Verfahren nach einem der Ansprüche 1 und 2, dadurch gekennzeichnet, daß das Enzym Prozym 6 *Aspergillus* (Amano Pharmaceutical Ltd.) ist.

**4.** Verfahren nach einem der Ansprüche 1 und 2, dadurch gekennzeichnet, daß das Enzym Acetonpulver vom Schweinepankreas Typ I (Sigma) ist.

**Claims**

**1.** Process for the preparation of alkyl (2*R-trans*)-2-piperidinecarboxylates of formula (1)

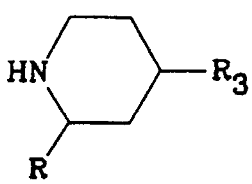

(1)

in which

R represents a ($C_1$-$C_4$) alkoxycarbonyl group and
$R_3$ represents a ($C_1$-$C_4$) alkyl group,

which process is characterized in that an alkyl 2-piperidinecarboxylate in the form of a racemate of trans configuration is reacted with an enzyme which specifically degrades the (2*S-trans*) enantiomer, leaving the (2*R-trans*) enantiomer largely intact.

**2.** Process according to Claim 1, characterized in that the enzyme is chosen from the lipase AY of *Candida rugosa* (Amano Pharmaceutical Ltd.), the lipase N of *Rhizopus* (Amano Pharmaceutical Ltd.), lipase Mucor (Amano Pharmaceutical Ltd.) or, prozyme 6 *Aspergillus* (Amano Pharmaceutical Ltd.), protease *Aspergillus oryzae* (Sigma), sheep pancreas acetone powder (Sigma) and porcine: type I pancreas acetone powder (Sigma).

**3.** Process according to either of Claims 1 and 2, characterized in that the enzyme is prozyme 6 Aspergillus (Amano Pharmaceutical Ltd.).

**4.** Process according to either of Claims 1 and 2, characterized in that the enzyme is porcine: type I pancreas acetone powder (Sigma).